# EUROPEAN PATENT SPECIFICATION

(11) **EP 2 906 157 B1**
(45) Date of publication and mention of the grant of the patent: **05.08.2020**
(21) Application number: 13812101.7
(22) Date of filing: 10.10.2013
(51) Int. Cl.: A61F 7/00, A61F 7/02

(54) **A THERAPEUTIC COLLAR**
THERAPEUTISCHE HALSKRAUSE
COLLIER THÉRAPEUTIQUE

(30) Priority: 10.10.2012 IT MO20120246
(43) Date of publication of application: 19.08.2015
(73) Proprietor: Neuron Guard S.r.l., 41124 Modena (IT)
(72) Inventor: GIULIANI, Enrico, I-41124 Modena (IT)
(74) Representative: Bergamini, Silvio
(86) International application number: PCT/IB2013/059262
(87) International publication number: WO 2014/057450

(56) References cited:
- WO-A1-2006/126059
- WO-A1-2011/118802
- WO-A2-2006/110405
- CZ-A3- 20 100 645
- DE-A1-102005 044 402
- DE-U1-202011 101 309
- JP-A- 2010 082 427
- JP-A- 2011 083 498
- US-A1- 2005 149 153
- US-A1- 2010 198 322
- US-A1- 2012 143 110

## Description

### Field of the invention

The invention relates to a therapeutic collar, preferably of disposable type, and generally used to cool particular zones of the patient's neck, to induce hypothermia in the circulatory system that supplies the brain.

### Background art

Collars are already known, which are typically used for rescuing severe trauma patients.

These collars have a structure that immobilizes or restricts the movements of the head relative to the spine, such that no externally-induced or patient-induced hazardous movements might endanger the overall patient's safety.

In practice, prior art collars inhibit or limit head movements, e.g. prevent reclination or upward rotation of the head beyond a given angle relative to the spine, and avoid crush or sprain injuries to vertebrae and cervical nerves, due to external impact trauma or a cervical disease.

Typically, these collars are used on patients involved in road accidents, to prevent movements during transportation to hospital or during later convalescence from further worsening any injury suffered due to the accident.

Typically, the structure of prior art collars is composed of two semi-annular portions which are mutually articulated to open apart for application to the neck, and to close for maintaining the correct position of the neck and head relative to the spine.

Collars are also known that have a semi-rigid structure, typically made of synthetic foam material, to act as transition supports from rigid-collar therapy to the end of the therapy.

With time, these collars have been also used in other forms of therapy, such as those for thermal treatment of the circulatory system of the neck, that supplies blood to the brain.

In practice, apertures are formed in prior art collars, namely in the front Section thereof, for introduction and stable but removable positioning of corresponding cooling elements, which have been previously placed and maintained in a refrigerating unit, and release cold during use and which are located at the carotid artery, i.e. the artery that supplies blood to the brain and extends through the front area of the neck, when collars are closed around the neck.

A collar of this type is known from Patent Application WO2012/058427.

This Patent Application teaches an "Immobilization collar with cooling elements and method of using the same".

The collar is actually a cervical immobilizer that has an annular support structure having an axial length and at least two support structures.

The collar has a front opening that may be closed by a door having a pressure member on the inner neck-facing surface.

This pressure member presses a cooling element against the front portion of the patient's neck, which cooling element is inserted between the collar and the neck through the front opening.

A "Device for induction of hypothermia is known from patent CZ 20100645A3.

According to this patent, the device consists of a cooling block which is connected to a Peltier module on one side, ad to a secondary cooling system via a connecting element on the other side.

A cooling element I s connected to the Peltier module on the side of alive tissue.

The cooling element is then linked to a contact element and it can be thermally insulated on the outside with insulation.

A dismountable joint is attached to the secondary cooling system or the supporting element or the cooling element and enables the individual cooling blocks to be interconnected.

After the device is attached to the tissue which is to be cooled down, the joint ensures the best possible contact between the cooled tissue and the contact element subsequently, the device for induction of hypothermia is attached via a clamp so that it is in desired contact with the cooled tissue.

The clamp ensures ideal contact conditions between the live tissue and the contact element.

The clamp is part of the secondary cooling system or supporting element or the cooling element.

The secondary cooling system works on the basis of heat transfer away from the warm side of the Peltier module, i.e. the cooling block, by means of fluid or air.

The above described prior art suffers from certain drawbacks.

A first drawback is that prior art collars that are used for first rescue of patients have a rigid structure, which is specially designed to inhibit any movement of the neck and spine in the proximity of the cervical vertebrae, when this part of the body suffers from a traumatic injury or a bone disease.

A second drawback is that the cooling elements that are used therein have a temporary effect that progressively decreases with time, especially because they are taken out of the refrigerator and placed in contact with the warm epidermis of the neck.

Therefore, after cyclic time intervals, the cooling elements must be replaced to maintain a desired low temperature value, according to the therapeutic needs of patients.

The replacement of these cooling elements is considerably uncomfortable for patients that already suffer from normally painful diseases, as it requires manipulation of collars by the operator, with the risk of causing movements that are hazardous for the vertebrae and might affect their physical recovery.

Furthermore, in case of diseases that are not caused by head trauma, but by cerebral anoxia or hypoxia, e.g. due to a cardiac arrest, the use of these prior art collars is not appropriate, as it immobilizes the patient and hinders treatment or makes therapy useless. WO2011/118802, JP2011083498 and JP2010082427 refer to additional patent documents relevant for the present invention.

### Disclosure of the invention

It is an object of the invention to improve the prior art.

Another object of the invention is to provide a therapeutic collar of disposable type, that allows constant thermal treatment of the neck zone with the arteries that supply the brain for as long as desired, e.g. by decreasing its temperature to a desired value to induce hypothermia and preserve for a given time the cerebral functions of a patient after cerebral anoxia or hypoxia or other serious injuries of the cranial or encephalic region, both during first rescue operations and during the following therapies at the hospital.

Another object of the invention is to provide a therapeutic collar that can be interfaced with a control unit, for controlling and adjusting the thermal conditions to be provided in contact with the patient's neck.

The invention provides a therapeutic collar as defined in claim 1.

The invention achieves the following advantages:
- it provides a therapeutic collar that is disposable, easily wearable and comfortable for patients;
- it provides a therapeutic collar that can maintain the neck of a patient at a desired and substantially constant temperature, for a desired and extended interval of time, without any degradation of thermal treatment;
- it provides a therapeutic collar that requires no displacement of the patient to prepare the required thermal treatment.

### Brief description of the drawings

Further features and advantages of the invention will be more readily apparent upon reading of the detailed description of a preferred non-exclusive embodiment of a therapeutic collar, which is shown as a nonlimiting example by the annexed drawings, in which:
FIG. 1 is a perspective schematic view of a therapeutic collar in the closed configuration;
FIG. 2 is a front schematic view of the collar of Figure 1 in a totally open configuration, providing a view of its interior;
FIG. 3 is a broken cross-sectional view of a portion of the collar of Figure 1, as taken along a plane III-III of Figure 2;
FIG. 4 is a highly schematic view of the head and neck of a patient that wears the therapeutic collar of the invention;
FIG. 5 is a highly schematic top view of a network of blood vessels, known as Circle of Willis, which supplies blood to the brain or encephalic region of a human being;
FIG. 6 is a highly schematic perspective and phantom view of a second arrangement of the collar;
FIG. 7 is a small-scale schematic view of a possible connection arrangement between the cooling units of the collar of Figure 6;
FIG. 8 is a schematic view of a collar according to the present invention with a second connection arrangement between the cooling units of the collar of Figure 6.
FIG. 9 is a schematic cross-sectional view of the therapeutic collar of the invention, as taken along a plane IX-IX of Figure 8.

### Detailed description of one preferred embodiment

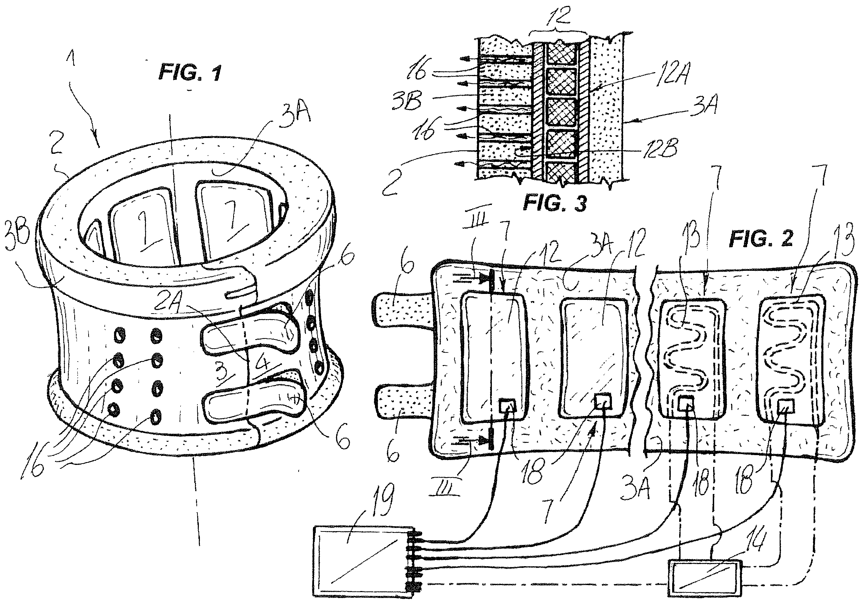

Referring now to Figures 1 to 5, numeral 1 generally designates a therapeutic collar, preferably but without limitation of disposable type, which comprises an annular body 2 having a longitudinal axis "A" and has a front cut to define a dividing plane 2A that divides it into two sections 3 and 4, which may be elastically opened apart to allow the collar 1 to be worn around the neck 40 of a patient "P" or to be removed.

As mentioned above, the collar 1 is preferably made of a disposable and flexible material and the two sections 3 and 4 are maintained in the closed state during use, using closure devices that are known to the skilled person, such as tear strips 6.

Cooling means, generally referenced 7, are arranged in the annular body 2 such that, when the collar 1 is closed around the neck 4, they are located at the carotid 8, vertebral 9 and median 15 arteries, that extend along the neck 40 and supply blood to the cranial-encephalic region (brain 10, cerebellum 11, etc.).

As shown in Figure 2, the cooling means 7 may comprise a plurality of Peltier cells 12, which are placed in side-by-side relationship until they cover substantially the entire inner surface 3A of the collar 2.

Alternatively, the cooling means 7 may comprise a plurality of serpentine-shaped tubes 13 in which a temperature-controlled liquid flows, which liquid is typically cooled to a predetermined temperature and is supplied by a pump unit 14.

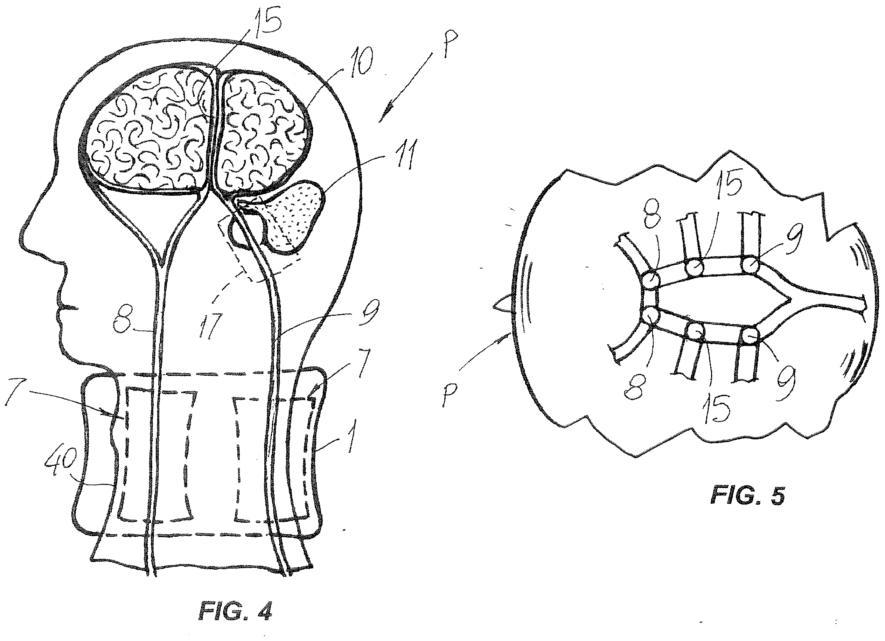

The skilled person will understand that other solutions may be envisaged to make the cooling means 7, as long as the latter are all positioned on the inner surface 3A of the collar 1, at the carotid arteries 8, the vertebral arteries 9 and the media arteries, referenced 15 (see Figure 5 in particular), to provide a controlled hypothermia state in the blood that flows therethrough, and hence in the entire brain or encephalic region.

In the arrangement of the collar 1 of Figures 1 and 3, if the cooling means 7 consist of Peltier cells 12, apertures 16 are provided for radiating out the heat generated by the heat transfer surfaces thereof facing opposite to the neck 40 when the collar 1 is applied thereto.

Peltier cells 12 are known to operate by creating two heat transfer surfaces, namely a cold surface 12A, in this case facing the neck 40 of the patient "P", and an opposite and parallel heat-generating surface 12B which, in the specific application of the collar 1, must be discharged through the apertures 16.

Furthermore, the material that forms the outer part of the annular body 2, which is referenced 3B in Figure 3, is of the thermo-insulating type, whereas the material that forms the inner surface 3A is thermally conductive, to facilitate hypothermic exchange with the neck 40.

Irrespective of whether the cooling means are in the form of Peltier cells 12 or serpentines 13, a connection is provided with temperature sensors 18 connected thereto and to a control unit 19 for the received data, which is designed to change and maintain the temperatures of the cooling means 7 constant, as needed.

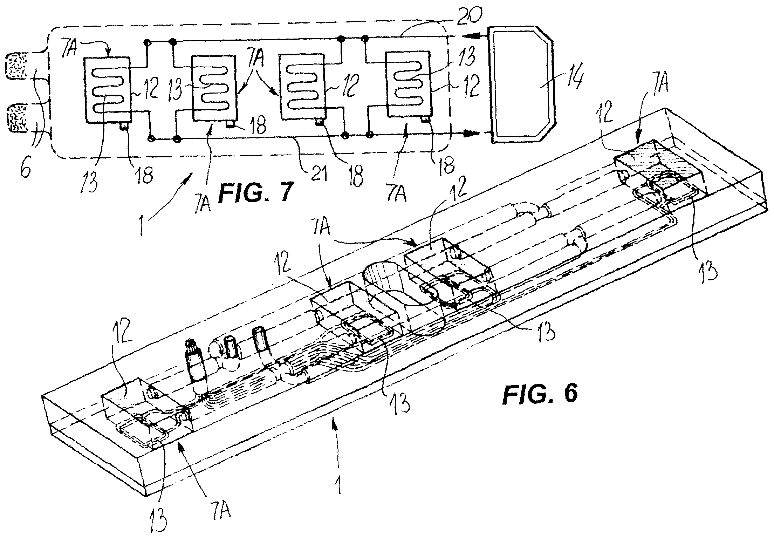

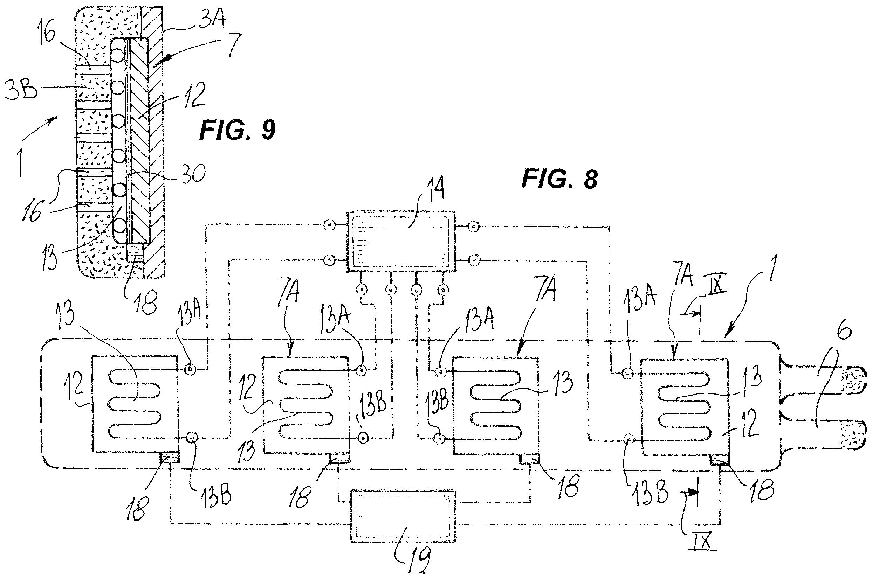

Figures 6 to 7 show a further possible arrangement of the collar 1. Figures 8,9 show an embodiment of the present invention.

In this embodiment, the cooling means 7 comprise a plurality of cooling units 7A, each consisting of the combination of a Peltier cell 12 and a serpentine 13, in close contact with each other, with a layer of conductive gel 30 only interposed therebetween, preferably a gel containing metal salts.

The serpentines 13 have respective inlet 13A and outlet 13B end pairs for receiving and discharging cooling fluids.

Each end pair 13A and 13B are directly connected to the pump unit 14, as shown in Figure 8.

The units 7A are also equipped with temperature sensors 18, which are in turn connected with the control unit 19 that receives and analyzes the data received from the sensor 18 and automatically accordingly controls the temperatures of the cooling fluids.

It shall be particularly noted that, the temperature changes of the cooling fluids are negligible as compared with the case in which the ends 13A and 13B are connected in series.

In Figure 4, numeral 17 schematically designates the center that controls the vital functions of the patient "P", which is located in the occipital region, close to the cerebellum 11 and that is maintained in a hypothermic state by the collar 1, if required, to avoid brain damage in case of hypoxia or anoxia, irrespective of the cause of these states in the patient "P".

The invention has been found to fulfill the intended objects.

The invention as conceived is susceptible to changes and variants within the scope of the appended claims.

## Claims

1. A therapeutic collar (1) designed to be fitted around the neck (40) of a patient (P) comprising:
- A flexible body (2) having in a worn configuration an annular shape and having a longitudinal axis (A), said body (2) comprising two end sections (3, 4) that can be parted reciprocally and defining a front section and a rear section and two side connecting zones;
- A dividing plane (2A) between said end sections (3, 4);
- A closing and opening device (6) of said two end sections (3, 4);
- A reciprocally coaxial inner wall (3A) and an outer wall (3B) of said body (2); said body (2) further comprising cooling means associated to said inner wall (3A) **characterized in that** said cooling means comprise a plurality of cooling units (7A), each consisting of the combination of a Peltier's cell (12) and a serpentine tube (13) in close contact with each other, with a layer of heat conductive gel (30) interposed therebetween, said serpentine (13) having respective inlet (13A) and outlet (13B); each cooling unit (7A) being further connected to a pumping group (14) and a control unit (19) through a respective connection which is independent from connections of other cooling units.

2. The therapeutic collar as claimed in claim 1, wherein said cooling means comprise constant cooling means (7).

3. The therapeutic collar as claimed in claim 1, wherein said conductive gel comprises a gel containing metal salts.

4. The therapeutic collar as claimed in anyone of preceding claims, wherein said body (2) comprises heat removal means (16) coming from said Peltier's cells (12) toward the outside.

5. The therapeutic collar as claimed in anyone of preceding claims, wherein said cooling means (7) are associated to said inner wall (3A) in contact zones wherein carotid arteries (8), vertebral arteries (9), middle arteries (15) pass.

6. The therapeutic collar as claimed in anyone of preceding claims, wherein it comprises an outer thermo-insulating portion (3B) and an inner thermo-conductive portion (3A).

7. The therapeutic collar as claimed in claim 1, wherein said dividing plane (2A) is parallel to said longitudinal axis (A).

8. The therapeutic collar as claimed in anyone of preceding claims, wherein each of said cooling units (7A) comprises automatic temperature control sensing means (18).

## Patentansprüche

1. Therapeutische Halskrause (1), die konstruiert ist, um um den Hals (40) eines Patienten (P) angelegt zu werden, umfassend:
- einen flexiblen Körper (2), der in einer getragenen Konfiguration eine Ringform aufweist und eine Längsachse (A) aufweist, wobei der Körper (2) zwei Endteile (3, 4) umfasst, die jeweils geteilt werden können und einen vorderen Teil und einen hinteren Teil und zwei seitliche Verbindungsbereiche definieren;
- eine Trennebene (2A) zwischen den Endteilen (3, 4);
- eine Schließ- und Öffnungsvorrichtung (6) der zwei Endteile (3, 4);
- eine gegenseitig koaxiale Innenwand (3A) und eine Außenwand (3B) des Körpers (2);
wobei der Körper (2) weiter der Innenwand (3A) zugeordnete Kühlmittel umfasst, **dadurch gekennzeichnet, dass** die Kühlmittel eine Vielzahl von Kühleinheiten (7A) umfassen, wobei jede davon aus der Kombination einer Peltier-Zelle (12) und eines Schlangenrohrs (13) in engem Kontakt untereinander mit einer dazwischenliegenden Lage wärmeleitenden Gels (30) besteht, wobei die Schlange (13) jeweils Einlass (13A) und Auslass (13B) aufweist;
wobei jede Kühleinheit (7A) weiter mit einer Pumpgruppe (14) und einer Steuerungseinheit (19) über eine jeweilige Verbindung verbunden ist, die von Verbindungen anderer Kühleinheiten unabhängig ist.

2. Therapeutische Halskrause nach Anspruch 1, wobei die Kühlmittel konstante Kühlmittel (7) umfassen.

3. Therapeutische Halskrause nach Anspruch 1, wobei das leitende Gel ein Gel umfasst, das Metallsalze enthält.

4. Therapeutische Halskrause nach einem der vorstehenden Ansprüche, wobei der Körper (2) Wärmeableitungsmittel (16) umfasst, die aus der Peltier-Zelle (12) nach außen kommen.

5. Therapeutische Halskrause nach einem der vorstehenden Ansprüche, wobei die Kühlmittel (7) der Innenwand (3A) in Kontaktbereichen zugeordnet sind, wobei Hauptschlagadern (8), Wirbelarterien (9), mittlere Arterien (15) durchgehen.

6. Therapeutische Halskrause nach einem der vorstehenden Ansprüche, wobei sie einen äußeren wärmeisolierenden Abschnitt (3B) und einen inneren wärmeleitenden Abschnitt (3A) umfasst.

7. Therapeutische Halskrause nach Anspruch 1, wobei die Trennebene (2A) parallel zu der Längsachse (A) ist.

8. Therapeutische Halskrause nach einem der vorstehenden Ansprüche, wobei jede der Kühleinheiten (7A) Abtastmittel zur automatischen Temperatursteuerung (18) umfasst.

## Revendications

1. Collier thérapeutique (1) conçu pour être ajusté autour du cou (40) d'un patient (P) comprenant :
- un corps souple (2) possédant dans une configuration portée une forme annulaire et possédant un axe longitudinal (A), ledit corps (2) comprenant deux sections d'extrémité (3, 4) qui peuvent être séparées de manière réciproque et définissant une section avant et une section arrière et deux zones de raccordement latérales ;
- un plan de division (2A) entre lesdites deux sections d'extrémité (3, 4) ;
- un dispositif d'ouverture et de fermeture (6) desdites deux sections d'extrémité (3, 4) ;
- une paroi interne réciproquement coaxiale (3A) et une paroi externe (3B) dudit corps (2) ;
ledit corps (2) comprenant en outre des moyens de refroidissement associés à ladite paroi interne (3A) **caractérisé en ce que** lesdits moyens de refroidissement comprennent une pluralité d'unités de refroidissement (7A), constituées chacune par la combinaison d'une cellule de Peltier (12) et d'un tube en serpentin (13) en contact étroit entre eux, avec une couche de gel conducteur de chaleur (30) intercalée entre eux, ledit serpentin (13) possédant une entrée (13A) et une sortie (13B) respectives, chaque unité de refroidissement (7A) étant en outre raccordée à un groupe de pompage (14) et à une unité de commande (19) par le biais d'un raccordement respectif qui est indépendant de raccordements d'autres unités de refroidissement.

2. Collier thérapeutique selon la revendication 1, dans lequel lesdits moyens de refroidissement comprennent des moyens de refroidissement constant (7).

3. Collier thérapeutique selon la revendication 1, dans lequel ledit gel conducteur comprend un gel contenant des sels de métaux.

4. Collier thérapeutique selon l'une quelconque des revendications précédentes, dans lequel ledit corps (2) comprend des moyens d'évacuation de la chaleur (16) venant desdites cellules de Peltier (12) et allant vers l'extérieur.

5. Collier thérapeutique selon l'une quelconque des revendications précédentes, dans lequel lesdits moyens de refroidissement (7) sont associés à ladite paroi interne (3A) dans des zones de contact dans lesquelles des artères carotides (8), des artères vertébrales (9) des artères moyennes (15) passent.

6. Collier thérapeutique selon l'une quelconque des revendications précédentes, dans lequel il comprend une partie thermiquement isolante externe (3B) et une partie thermiquement conductrice interne (3A).

7. Collier thérapeutique selon la revendication 1, dans lequel ledit plan de division (2A) est parallèle audit axe longitudinal (A).

8. Collier thérapeutique selon l'une quelconque des revendications précédentes, dans lequel chacune des unités de refroidissement (7A) comprend des moyens de détection de commande automatique de température (18).
